# EUROPEAN PATENT APPLICATION

(11) **EP 4 144 355 A1**
(43) Date of publication of application: **08.03.2023**
(21) Application number: 22189330.8
(22) Date of filing: 29.06.2017
(51) Int. Cl.: A61K 33/00, A61P 17/02

(54) **GOLD PARTICLE FOR USE IN THERAPY TO PREVENT SCAR FORMATION**

(30) Priority: 29.06.2016 EP 16176880
(62) Divisional of application: 17178814.4
(71) Applicant: ReGold ApS, 9640 Farsø (DK)
(72) Inventor: DANSCHER, Gorm, Aarhus (DK)
(74) Representative: AWA Denmark A/S

(57) **Abstract**

A gold particle having at least one cross-section in the range of 20 -1000 µm and having a purity greater than 99.00% w/w, preferably 99.99% w/w for use in treatment of a wound to minimize scar formation and to prevent abnormal scar formation. A composition comprising at least one gold particle having at least one cross-section in the range of 20 - 1000 µm and having a purity greater than 99.00% w/w, preferably 99.99% w/w for use in treatment of a wound to prevent abnormal scar formation, wherein the composition further comprises hyaluronic acid or any other physiologically acceptable carrier. A method of treatment of a wound of an animal or human patient to prevent or reduce scar formation wherein a dose of at least one gold particle having at least one cross-section in the range of 20 - 1000 µm and having a purity greater than 99.00% w/w, preferably 99.99% w/w is applied into the wound in contact with immune cells of the patient, thereby facilitated to release free gold ions by dissolucytosis.

## Description

The present invention relates to gold particles having a cross-section in the range of 20 - 1000 µm and having a purity greater than 99.00% w/w, preferably 99.99% w/w for use in treatment of a wound for prevention of excessive scar formation and to a method of treatment of wounds, sub epithelial scars resulting from use of fillers, or paradentosis.

### Background

Scar formation results from the biological process of wound repair in the skin and other tissues of the body. With the exception of very minor lesions, every wound (e.g., after an accident, a disease, or surgery) results in some degree of scarring. An exception to this is animals with complete regeneration, which re-grow tissue without scar formation.

Wounds naturally heal by themselves but depending on the severity of the wound, care may be needed such as halting the bleeding, treating infections as well as improve the healing process. Not all wounds heal as easy as others, and some wounds may even cause secondary complications such as infections, and in even worse cases non healing wounds could become chronic wounds, such as venous ulcers or pathological scarring such as keloid scars. Deciding on a treatment depends on the type of wound that a person has sustained. Varying from infections to burns, wound care is a priority in saving the limb, extremity, or life of a person. More severe wounds like diabetic ulcers, decubitus ulcers, and burns, require sterile, or clean dressings and wound care.

The process of wound healing is a complex interplay of cells, mediators and cytokines, which is not yet fully understood. The healing process starts off with clotting and recruitment of inflammatory cells and then proceeds to a highly proliferative state, which is characterized by deposition of granulation tissue and neovascularization. In general, the process of wound healing is divided into hemostasis (blood clotting), inflammation, proliferation (growth of new tissue), and maturation (remodeling).

A natural part or result of the healing process is scarring. More specifically, scars are areas of fibrous tissue (fibrosis) that replace normal skin after injury.

Poor healing may contribute to scars that are obvious, unsightly or disfiguring. Even a wound that heals well can result in a scar that affects the appearance and even comfort. Scars may be raised or recessed, different in color or texture from surrounding healthy tissue or particularly noticeable due to their size, shape or location. Scars may not require medical treatment, but they can be disfiguring and have a serious effect on a person's self-image and self-esteem and quality of life.

Early and effective treatment of the wound can prevent the severity of the scarring that often follows. As of today no prescription drugs for the treatment or prevention of scars are available. Low-dose, superficial radiotherapy is sometimes used to prevent recurrence of severe keloid and hypertrophic scarring. Silicone dressings are commonly used in preventing scar formation and improving existing scar appearance Stavrou D, Weissman O, Winkler E, Yankelson L, Millet E, Mushin OP, Liran A, Haik J (October 2010). "Silicone-based scar therapy: a review of the literature". Aesthetic Plastic Surgery 34 (5): 646-51*.*

Post scarring it may be possible to reduce scarring by using chemical peels, filler injections, dermabrasion, laser treatment, or, scarring may be reduced by surgery. Furthermore, corticosteroid injections may help flatten and soften the appearance of keloid or hypertrophic scars *(*Roques C, Téot L (2008). "The Use of Corticosteroids to Treat Keloids: A Review". The International Journal of Lower Extremity Wounds 7 (3): 137-145*.).*

The methods for the reduction of scars described above are usually marginally effective, painful, expensive, and often produce further scars during the procedure. Hence, there remains a need for finding safe solutions for treating wounds and reducing scar formation.

### Summary

With this background it is the object of the invention to alleviate one or more of the problems of the prior art. Hence, in one aspect is provided gold particles having a cross-section in the range of 20 - 1000 µm and a purity greater than 99.00% w/w, preferably 99.99% w/w for use in therapy to prevent or reduce the formation of a scar during healing of a wound.

In the context of the present invention abnormal scar formation is to be understood as histopathologic changes in response to trauma. Inflammation is a normal part of the natural wound-healing process which occurs after the blood coagulation stages, and is important for the removal of contaminating microorganisms. If the inflammatory process is prolonged, however, the wound may heal by overproduction of collagens causing abnormal or excessive scarring or even worse the wound may enter into a chronic state and fail to heal. Gold particles or gold implants according to the invention may be used early in the process of wound healing in the prophylactic treatment of abnormal scar formation during healing of a wound.

In the context of the present invention, gold should be understood as pure gold which in this context is at least 99.00% w/w, preferably at least 99.9% w/w gold, such as 99.99% w/w gold.

Upon application or injection of gold particles of a certain size within the range of 20 - 1000 µm in cross-sectional diameter into the affected tissue e.g. the margin of a wound or along a sutured wound or into the inflamed gingiva, the local macrophages and other inflammatory cells attach themselves to the metallic gold surfaces. The macrophages produce an ultra-thin layer, denoted a dissolution membrane, within which the macrophages control the chemical milieu including releasing cyanide ions. In this way the macrophages dissolve the outmost surface of the gold implants, a well known process called dissolucytosis, whereby gold ions, bound in gold cyanide molecule are released into the dissolution membrane. It has now turned out that this application is effective in the prevention and/or treatment of scars.

The use of pure gold is important from a safety point of view as some of the amalgamated metals in alloys can be toxic to the tissue.

New research, has now shown that gold ions liberated from pure gold according to the invention by macrophages do not spread further away than about 500 microns from the locality from where the metallic gold was administered. This is important from a safety point of view and reflects the fact that the amount of gold ions released is very low excluding a general exposure to the organism, i.e. the bio-released gold ions stay local.

In the context of the present invention dissolucytosis is the term for an extracellular liberation of gold ions, from the surface of the gold particles. The dissolucytosis takes place within the dissolution membrane and is most likely caused by the capability of the macrophages to release cyanide ions and alter the oxygen tension and the pH in their vicinity (Larsen A, Stoltenberg M. & Danscher, G (2007) In vitro liberation of charged gold atoms. Au-tometallographic tracing of gold ions released by macrophages grown on metallic gold surfaces 128, 1-6 Histochem Cell Biol.; Ferre N, Claria, J (2006) New insight into the regulation of liver inflammation and oxidative stress. Mini Rev. Med. Chem. 6, 1321-1330.). The inflammatory cells (e.g. the macrophages) release cyanide into the dissolution membrane and the following chemical process occurs:

4Au + 8CN⁻ + 2H₂O + O₂ = 4[Au(CN)₂]⁻ + 4OH⁻

The complex ion aurocyanide Au(CN)₂⁻, which is a relatively stable ion, inhibits the lysosomal enzymes of inflammatory cells in the synovial tissue and decreases the number of inflammatory cells in situ.

Without the wish to be bound by any theory it is believed that this reduction of the local immune response allows the wound to heal without excessive scar formation and gingiva suffering from paradentosis to repair

The molecular mechanisms of gold also entail decreased levels of pro-inflammatory mediators like interleukins, tumor necrosis factor alpha (TNFα) and eicosanoids, as well as inactivation of phagolysosomal enzymes, Inhibitor of kappa B (IκB) kinase and nuclear factor-kappa B (NF-B) activity.

It is herein disclosed, that injecting gold particles of a certain size with a cross-section in the range of 20 - 1000 µm, such as 20 - 75 µm, into the affected tissue of a wound aids in the healing of the wound, and reduces scar formation.

In a transplantation study of the ability of metallic gold to suppress inflammation locally it was noticed that metallic gold causes a pronounced decrease in the formation of granulation tissue (Danscher et al. 2006 unpublished observation*).* This observation was important for the realization of using gold in the prophylactic treatment of scars where overproduction of granulation tissue is a medical problem (e.g. keloid) as well as in prevention of scar formation in plastic surgery.

In addition, for the prevention and reduction of granulation tissue, gold particles with a cross-section diameter in the range from 20-1000 microns, according the invention may be used in prophylactic treatment of keloid, hypertrophic or atrophic scars and for healing of jar bone and gingiva resulting from e.g. paradentosis. In addition to an anti-inflammatory effect, metallic gold has further proven to have a weak bactericidal and analgesic effect, thus causing a pain relief in patients susceptible to abnormal scarring when being treated with gold particles according to the invention. The analgesic effects might be caused by the mast cells being unable to release histamine and thereby causing a reduction in local oedema.

The cross-sectional diameter of the gold particles may vary according to the size and form of the wound and may be any number from 20 and up to 1000 µm.

The effect of using gold particles or gold implants having a cross-section in the range of 20 - 1000 µm instead of smaller gold particles, such as gold nano-particles, or gold salts usually applied in therapy, is that the > 20 micron sized gold particles will not migrate from the position where they are applied and thus, they are kept in proximity to the wound where the bio-release of gold ions is needed to reduce inflammation. In this way the healing process is improved and scar formation reduced.

Gold particles smaller than 20 µm, are, as mentioned, readily phagocytosed and removed from the location by phagocytes. They will not be subjected to dissolucytotic release of gold ions.

Larger gold particles on the other hand, that being, larger than 1000 µm will not bring about more gold ions because the gold surface relative to the mass of gold is too little. On top of that, the expenses of gold will be unnecessarily high. The pure gold particles of the present invention may be of any shape, however, at least one cross-section must be larger than 20 µm. Preferred shapes of the gold particle could for instance be hollow or solid, beads, polygons, rods, curled up wires, coils, flakes, shavings sheets, rings, discs, or biconcave discs. For example as described in US 7,655,261 B2 using gold for treatment of inflammatory diseases the gold particle may be hollow with a cross section of 100- 300 micron (µm). As the dissolucytosis process will occur on the surface of the gold implants, it is important that a large surface area to volume ratio be used in order to maximize the amount of bio-released gold ions (Danscher 2002).

In one embodiment, the cross-section of the gold particle is in the range of 20-40 microns.

In one embodiment, the cross-section of the gold particle is formed as a micron ball gold thread. The advantage of forming the gold particle as a gold thread is to increase the surface area relative to amount of gold.

In one embodiment, the gold particle is administered in an effective amount into the wound accessible for contact with immune cells of a patient. An effective amount in the context of the present invention is typically application of 10 mg/mL as further detailed below.

In another embodiment according to the invention the scar is formed from granulation tissue.

In yet another embodiment according to the invention the gold particles are for use in the treatment of a wound to prevent or reduce keloid, hypertrophic or atrophic scars.

In an embodiment of the invention the gold particles are used in treatment of chronic or diabetic wounds.

In a further embodiment of the invention, the gold particles are used in therapy to prevent or reduce the formation of scar during healing of a wound, wherein the scar is a psoriasis scar

In an embodiment of the invention the gold particles are used to prevent or reduce the formation of a scar during healing of a wound, wherein the scar is a sub epithelial scar resulting from use of fillers, or paradentosis.

In the context of the present invention fillers should be understood as an injectable filler that may be injected into the soft tissue of a patient to reduce the formation of scars. The filler is typically a formulation comprising natural or synthetic compounds, preferably sugar molecules, fats, hyaluronic acid, collagens, polymers including poly(methyl methacrylate) (PMMA), biopolymers or a combination thereof.

In an embodiment of the invention the gold particles are for use in therapy to prevent or reduce the formation of a scar during healing of a wound in a patient in need thereof.

In another embodiment, the gold particles are for use in therapy to reduce scar formation during local bone restoration. An example of local bone restoration may be dental restoration such as a tooth filling. By tooth filling is meant that teeth may be filled with gold, silver amalgam, or tooth-colored plastic materials or composite resin fillings.

Another aspect of the invention relates to a composition comprising at least one gold particle having a cross-section in the range of 20 - 1000 µm, preferably 20-40 microns and having a purity greater than 99.00% w/w, preferably 99.99% w/w and optionally hyaluronic acid or any other physiologically carrier that makes suspension of the particles possible for use in the treatment of wounds to prevent scar formation. A composition comprising hyaluronic acid is favorable as a formulation for injection of the gold particles into the affected tissue of a wound to be treated due to its high carrying capacity as described in US2014/0194852. A high carrying capacity is advantageous as the gold particles are maintained in suspension for longer and thereby provide a more homogeneous distribution of the gold particles in the composition and ultimately in the tissue of the wound. It is contemplated, however, that numerous additional carriers may be produced and used for the suspension of the gold particles according to the invention such as sterile water or phosphate-buffered saline or any other formulation suitable for injection. Additional components of the composition may be a local anaesthetic such as lidocain.

Yet another aspect of the present invention relates to a method of treatment of a wound of an animal or human patient to prevent scar formation, wherein an effective amount of gold particles having a cross-section in the range of 20 - 1000 µm and having a purity greater than 99.00% w/w, preferably 99.99% w/w is applied into the wound accessible for contact with immune cells of the patient, thereby said gold particles are facilitated to release free gold ions by dissolucytosis.

In an embodiment of all aspects, the gold particles are administered directly into the base of the wound and/or administered by injection into the affected tissue of the wound. In a further embodiment, the wound originates from a surgical incision. In this embodiment the method and treatment comprises the steps of applying the gold particles or composition according to the invention to the exposed tissue of the surgical incision in an effective amount of gold particles having a cross-section in the range of 20 - 1000 µm and having a purity greater than 99.00% w/w, preferably 99.99% w/w, the composition is administered along said walls of the incision and into said walls i.e. after suturing the gold implants are injected along and/or under and/or into the sutured edge of the wound.

The gold particles and composition according to the invention is further contemplated to be used in cosmetic surgery including corrective surgery, emergency surgery, reconstructive surgery and plastic surgery including use of fillers or to reduce scar formation related to wounds arising from pimples, minor cuts and burns, piercing, or any other abrasion of the skin.

Upon application or injection of gold particles having a cross-section in the range of 20 - 1000 µm and having a purity greater than 99.00% w/w, preferably 99.99% w/w into walls of the wound, the gold particles should preferably be distributed evenly along the intersecting wound edges after finishing the suturing. In the case of using fillers the gold particles are mixed into the filler and will reduce or prevent formations of sub epithelial condensation of scar tissue.

The treatment may advantageously be initiated immediately after the lesion of the skin occurs or as soon as possible thereinafter. The sooner in the process of wound healing the treatment according to the invention can be initiated the better the effect of preventing scarring.

### Figures

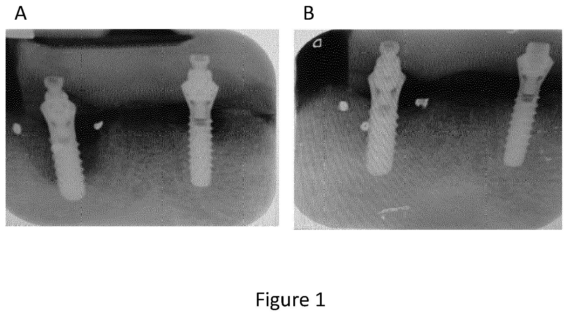

Figs. 1a and b show X-ray photographs of a patient with an implant just after implantation and after 8 months

### Detailed description of the invention

When used herein, the term gold or gold particle is to be understood as pure gold, not a solute such as gold ions in a medium.

When used herein, the term gold or gold particle is used interchangeable with gold implant.

When used herein, the terms "wound", "ulcer", "lesion" or "trauma" is to be understood as an injury to the subject, which has left the injured tissue or organ discontinued, such as an injury of the skin and underlying tissues, by a cut, rip, incision etc. It is contemplated that these terms may cover other kinds of injuries requiring healing or restoration of tissue.

When used herein, the term granulation tissue is to be understood as new connective tissue and tiny blood vessels that form in relation to wound healing.

When used herein, the term keloid or keloidal scar is to be understood as formation of an abnormal type of scar which, depending on its maturity, is composed mainly of either type III (early) or type I (late) collagen. It is a result of an overgrowth of granulation tissue (collagen type III) at the site of a healed skin injury which is then slowly replaced by collagen type I. Keloids are firm, rubbery lesions or shiny, fibrous nodules, and can vary from pink to the color of the patient's flesh or red to dark brown in color. A keloid scar is benign and not contagious, but sometimes accompanied by severe itchiness, pain, and changes in texture.

When used herein, the term hypertrophic scar is to be understood as formation of an abnormal type of scar which is a cutaneous condition characterized by deposits of excessive amounts of collagen which gives rise to a raised scar, but not to the degree observed with keloids. Like keloids, hypertrophic scars form most often at the sites of pimples, body piercings, cuts and burns. They often contain nerves and blood vessels. They generally develop after thermal or traumatic injury that involves the deep layers of the dermis. When a normal wound heals, the body produces new collagen fibers at a rate which balances the breakdown of old collagen. Abnormal scars such as hypertrophic scars are red and thick and may be itchy or painful.

When used herein, the term atrophic scar is to be understood as formation of an abnormal type of scar which is a sunken recess in the skin, which has a pitted appearance. These are caused when underlying structures supporting the skin, such as fat or muscle, are lost. This type of scarring is often associated with acne, chickenpox, other diseases, especially Staphylococcus or MRSA infection, surgery, or accidents.

When used herein, the term chronic wound is to be understood as a wound that does not heal in an orderly set of stages and in a predictable amount of time the way most wounds do; wounds that do not heal within three months are often considered chronic. Chronic wounds seem to be detained in one or more of the phases of wound healing. For example, chronic wounds often remain in the inflammatory stage for too long. In acute wounds, there is an accurate balance between production and degradation of molecules such as collagen; in chronic wounds this balance is lost and the healing is impaired.

When used herein, the term 'gold thread micron ball' is a thread of pure gold rolled into a micron-sized ball. The gold tread may have any diameter, preferably 50 micron to ensure a large surface to gold ratio. The gold thread micron ball may be placed directly into the wound as a micro implant.

Gold particles according to the invention may be dispersed in a carrier substance at a concentration of at least 0.1 mg/ml of gold particles, preferred 1 mg/ml, more preferred 5 mg/ml and most preferred 10 mg/ml of gold particles. The amount of gold in a carrier substance varies with the size of the wound. Often 2 ml of gold in a carrier substance such as hyaluronic acid with a concentration of 10 mg/ml of gold is sufficient to cover the lining of a wound. In one embodiment 10 mg/ml of gold corresponds to approximately 72.000 gold particles per ml.

Treatment may be initiated at any time during the healing of a wound, however, the greatest effect of preventing or reducing scarring is when the gold is applied into the wound as soon as possible and at least before suture. One dose treatment is sufficient since the gold will remain for a lifetime in the tissue of the patient where it has been placed.

The following describes experimental research supporting the effect of the invention.

### Background example

Gold particles usable in the invention implanted into injured brain tissue of mice revealed that the dissolucytotic gold ions suppress inflammation and influence the healing process in several different ways.

20-45 µm gold particles were injected into the neocortex of mice before generating a cryo-injury. It was found that the released gold ions reduced microgliosis and neuronal apoptosis, inflicted a transient astrogliosis, and increased the neural stem cell (NSC) response (Larsen A, Kolind K, Pedersen DS, Doering P, Pedersen MO, Danscher G, et al. (2008), "Gold ions bio-released from metallic gold particles reduce inflammation and apoptosis and increase the regenerative responses in focal brain injury", Histochem. Cell Biol. 130(4), 681-92). Following unilateral treatment with gold particles, NSC stimulation with increased M-CSF expression was seen. The metallic gold treatment also significantly increased the expression of the growth factors VEGF, FGF, LIF and neurotrophin-4 (Pedersen MØ, Larsen A, Pedersen DS, Stoltenberg M, Penkova M (2009), "Metallic gold treatment reduces proliferation of inflammatory cells, increases expression of VEGF and FGF, and stimulates cell proliferation in the subventricular zone following experimental traumatic brain injury", Histol. Histopathol. 24(5):573-86; Pedersen MØ, Larsen A, Stoltenberg M, Penkowa M (2010), "Bio-released gold ions modulate expression of neuroprotective and hematopoietic factors after brain injury", Brain Res. 1307, 1-13). Furthermore, metallic gold has been found to reduce TNF-α expression, oxidative DNA damage and pro-apoptotic signals after experimental brain injury, while at the same time an increase in the expression of the neuroprotective proteins MTI was recorded (Pedersen MO, Larsen A, Pedersen DS, Stoltenberg M, Penkowa M (2009), "Metallic gold reduces TNFalpha expression, oxidative DNA damage and pro-apoptotic signals after experimental brain injury", Brain Res. 19, 1271-103-13).

The neuroprotective and regeneratory qualities of metallic gold were seen as a reduction of apoptotic cell death at both the early and the late phase, and NSE and TUNEL staining confirmed a gold dependant circumvention of apoptosis in neurons (Larsen et al. 2008).

The amount of gold injected into the neocortex of the mice was 5.4 mg pure gold while the amount of bio-released gold ions was so low that only the most sensitive quantitative analyses can trace such small amounts. The tool that has made it possible to observe the dissolucytotic activities leading to liberation of gold ions is the histochemical technique autometallography (AMG) (Danscher G (1981) "Localization of gold in biological tissue: A photochemical method for light and electron microscopy", Histochemistry 71, 81-88; Danscher G, Stoltenberg M (2006), "Autometallography (AMG): Silver enhancement of quantum dots resulting from (1) metabolism of toxic metals in animals and humans, (2) in vivo, in vitro and immersion created zinc-sulphur/zinc-selenium nanocrystals, (3) metal ions liberated from metal implants and particles", Prog. Histochem. Cytochem. 41, 57-139).

In conclusion, bio-liberated gold ions have been shown to possess pronounced anti-inflammatory, neuroprotective and neurostimulatory capacities in the mouse brain and as inflammation in the central nervous system (CNS) has been found to resemble the inflammatory processes taking place in other tissues.

### Example of the invention

In another example according to the invention a patient having a dental implant was treated for paradentosis by the insertion of gold implants, made up of a ball of a 50 micron thick wire of 99.99 % pure gold, having a cross-section of 1000 µm. The gold implants/particles were injected into the periodontium in proximity to the gold implant as shown in figure 1A. Figure 1B shows the position of the gold particles after 8 months. Neither the gold implants nor the dental implants had moved position in the period of time. A visible regrowth of bone had taken place and no scarring was to be seen.

Further embodiments of the invention are:
Embodiment 1: Use of at least one gold particle having a cross-section in the range of 20 - 1000 µm and having a purity greater than 99.00% w/w, preferably 99.99% w/w for the cosmetic treatment of scar formation during healing of a wound.
Embodiment 2: Use of at least one gold particle according to embodiment 1 in fillers for cosmetic treatment of sub epithelial scar formation.

### Items

Item 1: Gold particle having a cross-section in the range of 20 - 1000 µm and having a purity greater than 99.00% w/w, preferably 99.99% w/w for use in therapy to prevent or reduce the formation of a scar during healing of a wound.

Item 2: Gold particle according to item 1 having a cross-section in the range of 20 - 1000 µm for use in prophylactic therapy to prevent or reduce the formation of a scar during healing of a wound.

Item 3: Gold particle according to according items 1 or 2 for use in therapy to prevent or reduce the formation of a scar during healing of a wound, wherein the cross-section is in the range of 20-40 microns.

Item 4: Gold particle according to any of the preceding items 1 to 3, for use in therapy to prevent or reduce the formation of a scar during healing of a wound, wherein the gold particle is formed as a micron ball gold thread.

Item 5: Gold particle according to any of the preceding items 1 to 4 for use in therapy to prevent or reduce the formation of a scar during healing of a wound, wherein an effective amount of the gold particle is administered into the wound accessible for contact with immune cells of a patient.

Item 6: Gold particle according to any of the preceding items 1 to 5, for use in therapy to prevent or reduce the formation of a scar during healing of a wound, wherein the scar is formed from granulation tissue.

Item 7: Gold particle according to any of the preceding items 1 to 6, for use in therapy to prevent or reduce the formation of a scar during healing of a wound, wherein the scar is a keloid, a hypertrophic or an atrophic scar.

Item 8: Gold particle according to any of the preceding items 1 to 7, for use in therapy to prevent or reduce the formation of a scar during healing of a wound, wherein the wound is a chronic wound or a diabetic wound.

Item 9: Gold particle according to any of the preceding items 1 to 8, for use in therapy to reduce pain in patients susceptible to abnormal scarring.

Item 10: Gold particle according to any of the preceding items 1 to 8, for use in therapy to prevent or reduce the formation of a scar during healing of a wound, wherein the scar is a sub epithelial scar resulting from use of local bone restoration, or paradentosis.

Item 11: Gold particle according to any of the preceding items 1 to 10, for use in therapy to prevent or reduce the formation of scar during healing of a wound, wherein the wound is a psoriasis wound.

Item 12: Gold particle according to item 10, wherein the local bone restoration is a filling.

Item 13: Gold particle according to any of the preceding items 1 to 12, for use in therapy to prevent or reduce the formation of a scar during healing of a wound in a patient in need thereof.

Item 14: Composition comprising at least one gold particle having a cross-section in the range of 20 - 1000 µm and having a purity greater than 99.00% w/w, preferably 99.99% w/w and optionally any other physiologically acceptable carrier for use in therapy to prevent or reduce the formation of a scar during healing of a wound.

Item 15: Composition according to item 14, wherein the physiologically acceptable carrier is hyaluronic acid.

## Claims

1. Gold particle having a cross-section in the range of 20-1000 µm, preferably in the range of 20-40 microns, and having a purity greater than 99.00% w/w, preferably 99.99% w/w, for use in therapy to prevent or reduce the formation of a scar during healing of a wound.

2. Gold particle for use according to claim 1, wherein the therapy is a prophylactic therapy.

3. Gold particle for use according to claim 1 or 2, wherein the therapy is initiated immediately after a lesion of the skin occurs or as soon as possible thereinafter.

4. Gold particle for use according to claim any of the preceding claims, wherein the gold particle is administered directly into the base of the wound and/or administered by injection into the affected tissue of the wound.

5. Gold particle for use according to any of the preceding claims, wherein the gold particle is hollow.

6. Gold particle for use according to any of the preceding claims, wherein the gold particle is formed as a micron ball gold thread.

7. Gold particle for use according to any of the preceding claims, wherein an effective amount of the gold particle is administered into the wound accessible for contact with immune cells of a patient.

8. Gold particle for use according to any of the preceding claims, wherein the scar is formed from granulation tissue.

9. Gold particle for use according to any of the preceding claims, wherein the scar is a keloid, a hypertrophic or an atrophic scar.

10. Gold particle for use according to any of the preceding claims, wherein the wound is a chronic wound or a diabetic wound.

11. Gold particle for use according to any of the preceding claims, for use in therapy to reduce pain in patients susceptible to abnormal scarring.

12. Composition comprising at least one gold particle having a cross-section in the range of 20 - 1000 µm and having a purity greater than 99.00% w/w, preferably 99.99% w/w, and optionally any other physiologically acceptable carrier for use in therapy to prevent or reduce the formation of a scar during healing of a wound.

13. Composition for use according to claim 12, wherein the composition comprising at least one gold particle is administered into the wall of the wound by injection.

14. Composition for use according to claim 13 or 14, wherein the gold is dispersed in the physiologically acceptable carrier at a concentration of at least 0.1 mg/ml of gold particles, preferred 1 mg/ml, more preferred 5 mg/ml and most preferred 10 mg/ml of gold particles, optionally wherein the physiologically acceptable carrier is hyaluronic acid.

15. Gold particle having a cross-section in the range of 20-1000 µm, preferably in the range of 20-40 microns, and having a purity greater than 99.00% w/w, preferably 99.99% w/w, for use in therapy to prevent or reduce an inflammatory condition of the skin and underlying tissue of a subject, wherein the inflammatory condition is psoriasis.
